Europäisches Patentamt

European Patent Office

Office européen des brevets

(19)

(11) Publication number: **0 160 266 B1**

(12) **EUROPEAN PATENT SPECIFICATION**

(45) Date of publication of patent specification: **11.12.91**  (51) Int. Cl.⁵: **A61K 9/127**

(21) Application number: 85104930.4

(22) Date of filing: 23.04.85

(54) Liposome composition.

(30) Priority: 28.04.84 JP 86570/84

(43) Date of publication of application:
06.11.85 Bulletin 85/45

(45) Publication of the grant of the patent:
11.12.91 Bulletin 91/50

(84) Designated Contracting States:
**BE DE FR GB NL SE**

(56) References cited:
**WO-A-85/03640**

**CHEMICAL ABSTRACTS, vol. 97, no. 22, 29th November 1982, page 376, no. 188156k, Columbus, Ohio, US; M. MEZEI et al.: "Liposomes, a selective drug delivery system for the topical route of administration: gel dosage form"**

**CHEMICAL ABSTRACTS, vol. 99, no. 11, 12th September 1983, no. 84551k, Columbus, Ohio, US; S. BAUER et al.: "Hyperfiltration through crosslinked monolayers. II."**

**JOURNAL OF PHARMACEUTICAL SCIENCES, vol. 69, no. 11, November 1980, pages 1271-1273, American Pharmaceutical Associ-** ation, US; S. HARRIS et al.: "Preparation, characterization, and stability of new prostaglandin E2 gel for local administration"

(73) Proprietor: **TERUMO KABUSHIKI KAISHA trading as TERUMO CORPORATION**
**44-1, 2-chome, Hatagaya Shibuya-Ku Tokyo 151(JP)**

(72) Inventor: **Aoyagi, Juuro**
**9-11, 3-chome Hamadayama**
**Suginami-ku Tokyo(JP)**
Inventor: **Yamamoto, Yuichi**
**398, Yumisawa-cho Fujinomiya-shi Shizuoka-ken(JP)**
Inventor: **Akaike, Toshihiro**
**15-23, 4-chome Shimohoya Hoya-shi Tokyo(JP)**
Inventor: **Nozaki, Yasuhiro**
**2-302, Biwakomisoradainidanchi, 1-3 Misora-cho Ootsu-shi Shiga-ken(JP)**

(74) Representative: **Henkel, Feiler, Hänzel & Partner**
**Möhlstrasse 37**
**W-8000 München 80(DE)**

**Description**

This invention relates to a method of preparing a liposome composition and more particularly to a liposome composition having an excellent property of enabling a material enclosed in the liposome composition to be released into the living body at a satisfactory slow rate.

Liposome is generally defined to mean an extremely small closed cell having a bimolecular film structure and mainly prepared from phospholipid. It is proposed to apply the medicine-supporting liposome formulation or composition as means for the stable storage of ordinarily unstable medicines, the slow release for a medicine within a living body and the selective transportation of a medicine to a particular internal organ. Already known is a liposome formulation containing, for example, insulin, heparin or urokinase which is intended to ensure the stable storage of ordinarily unstable medicines or the slow release of a medicine within the living body. Also proposed is a liposome formulation containing, for example, ubidecarenone or cytosine arabinoside.

A phospholipid applied as a film material of the conventional medicine-supporting liposome formulation includes, for example, phosphatidylcholine, phosphatidylethanolamine, phosphotidylinosytol, phosphatidylserine and sphingomyelin which are derived from edd yolk, soybean and animal tissues, egg yolk lecithin and soybean lecithin which are a mixtures of the above-noted phospholipids, or synthetic lecithin such as dipalmitoyl lecithin and distearoyl lecithin. The above-mentioned phospholipids have a good affinity to the living body and is safely applicable as an oral formulation or parenteral formulation such as an injection material. However, the conventional liposome film material has an insufficient stability, and is easily broken in the in vitro or in vivo application, thereby undesirably giving rise to the leak of a medicine at one time.

It is accordingly the object of this invention to provide a liposome composition or formulation whose film structure can be stably maintained in both in vitro and in vivo applications, thereby enabling materials taken into the liposome to be stably administered into the living body.

To attain the above-mentioned object, this invention provides a liposome composition which comprises:

a cross-linked material having a three-dimensional network structure; and

a liposome physically entrapped within the network structure and enclosing a material which is to be administered into a living body. The obtained lipsome is held between each lattice which is the minimum unit consisting said network structure, and said lipsome has a particle size larger than the lattice.

The above-mentioned crosslinked material is preferred to have a hydrophilic group or groups and a high biocompatibility. Application of a high molecular material having a high biocompatibility offers the advantage that when administered into the interior of the living body, the material is little likely to be recognized as a foreign matter by the living body. Further, the crosslinking of the above-described crosslinked material should preferably be carried out by irradiation with radiation such as gamma rays.

This invention can be more fully understood from the following detailed description when taken in conjunction with the accompanying drawings, in which:

Figs. 1 to 3 are graphs showing the comparison of the stability of the liposome composition prepared by this invention with that of the controls; and

Fig. 4 is a graph showing the comparison of the stability of the liposome composition of the invention when administered into the vein of a rat with that of the control.

The present inventors have conducted earnest studies to develop a liposome composition (or preparation or formulation) by a method of preparing a liposome composition including a cross-linked material having a three-dimensional network structure, and a liposome enclosing a medicine to be administered into a living body, said method comprising:

preparing an aqueous solution of a cross-linkable material;

mixing a liposome enclosing a medicine with said aqueous solution; and

irradiating the resulting mixture with gamma-rays, thereby cross-linking said cross-linkable material to have a three-dimension network structure and physically entrapping said liposome within said network structure.

This method enables a medical material taken into the liposome to be administered into a living body with a satisfactory slow rate. As a result, the present inventors have discovered that when a liposome containing a medical material to be administered into the living body is physically entrapped within a three-dimensional network structure portion (generally expressed as a matrix, latice or frame work; and hereinafter referred to as "matrix") of a crosslinked high molecular material, the matrix acts not only as a buffer (to prevent the liposome from directly contacting a body fluid to be broken in a short time), but also as a support capable of securely holding the liposome in the matrix. They have further discovered that when the high molecular material is crosslinked by irradiation with γ-rays to provide a matrix including a three-dimentional network structure of the crosslinked material, it is possible to render the high molecular material insoluble without damaging the liposome film and imparting toxicity thereto.

As used herein, the term "biocompatibility" is defined to mean that when administered into the living body, the liposome formulation has a satisfactory affinity to the living body and is substantially harmless thereto.

The high molecular materials used in this invention include polyacrylamide having an average polymerization degree of 500 to 3,000; poly(2-hydroxyethylmethacrylate); polymethacrylic acid; poly(N, N-dimethylaminoethylmethacrylate); polyethylene oxide; polyvinylalcohol; gelatin; polyvinylpyrrolidone; polysaccharides such as chitin; sodium alginate; collagen; fibrin; silicone; and collodion (preferably derivatives thereof). It is advised that the above-listed materials be used alone or in combination in the form of an aqueous solution having 3-20 W/V % concentration containing, if necessary, a crosslinking agent such as methylenebisacrylamide.

Among the above-mentioned aqueous solutions, the hydrogel is preferred. Since it contains a hydrophilic groups such as hydroxyl group, carbonyl group, and amino group, and possesses water-holding nature it has high biocompatibility, is little likely to be regarded as a foreign matter by the living body, and little tends to plug peripheral blood vessels and tissues. The hydrogel offers further advantages that since the high molecular material is surrounded by a large amount of structural water, and, even when dried by freezing, contracts itself in the network form, the liposome alone does not shrink upon drying and is not released from the network structure of the high molecular material.

As used herein, the matrix is defined to mean a substrate which is composed of the cross-linked high molecular materials and has a three-dimensional (steric) network structure. The network structure can be observed by an electron microscope. As used in this specification, the term "crosslinking" is construed in the broad sense, and includes not only the case where high molecular substances are chemically bonded together in the network structure, but also the case where the high molecular substances of the same type present a network structure by copolymerization or condensation polymerization among themselves. Also applicable to this invention is the crosslinking type which is based, as is well known, on the application of a crosslinking agent or a radiation such as gamma ray. The high molecular substances are insolubilized by the cross-linking and are not soluble in a common solvent. The crosslinked materials have a melting point higher than the substances before the crosslinking and have an increased strength. The crosslinked material has a high molecular weight, which can be recognized by measuring a viscosity average molecular weight or by gel permeation chromatography. Preferable high molecular materials are pharmaceutically acceptable (or biocompatible). (The above-listed high molecular substances are all possessed of satisfactory biocompatibility.)

For the present invention, it is preferred to carry out the formation of the aforesaid matrix, that is, the insolubilization, by radiation of gamma rays at a dose of 0.5 to 4 Mrad. This matrix-forming process offers the advantages that insolubility can be ensured without damaging the liposome film, but also eliminates the possibility of imparting toxicity to the living body as in the case of applying a polymerization-initiating agent. Particularly when a polymerization-initiating agent such as potassium persulfate or ammonium persulfate is applied, the drawback arises that the liposome film is damaged, or the liposome itself is broken.

The liposome is physically entrapped between the lattices (minimum units constituting the network structure) of the three-dimensional network structure of the matrix while holding a materials to be administered into the living body within the film thereof. Since the size of the liposome is larger than the lattice, the liposome entrapped within the lattices can not be removed therefrom. The liposome is entrapped particularly in that portion of the matrix which contains a hydrophilic group. Since the liposome can not escape from the interior of the matrix, it is assumed that the liposome film remains stabilized, enabling the material held in the liposome film to be released into the living body at a satisfactorily slow rate.

The material constituting the liposome film of this invention is provided by a lipid adapted for the formation of liposome. Phospholipid may be particularly cited as a material suitable for the formation of the liposome film. It is possible to apply any desired type of phospholipid such as natural phospholipids such as soybean lecithin and egg yolk lecithin, or synthetic lecithin or hydrogenated natural phospholipid. Since all natural phospholipids contain unsaturated fatty acid, it is more effective to apply hydrogenated phospholipid prepared by saturating the unsaturated fatty acid of the above-mentioned natural phospholipid with hydrogen. For the object of this invention, the synthetic phospholipid may be applicable, though expensive.

The phospholipid typically includes, for example, lecithin (phosphatidylcholine), phosphatidylethanolamine, phosphatidic acid, phosphatidyl inosytol, phosphatidylserine, phosphatidylcerol, sphingomyelin, and cardiolipin. The phospholipid further includes the types obtained by the ordinary hydrogenation of the above-listed materials. The particularly preferred types of the phospholipid include hydrogenated natural lecithin obtained by the hydrogenation of, for example, soybean lecithin, egg yolk lecithin, corn lecithin, cotton seed oil lecithin and rapeseed lecithin.

It is also possible to mix the liposome film material with a higher fatty acid. The preferred higher fatty acids include those which have 10 to 20 carbon atoms, for example, caprilic acid, lauric acid, myristic acid, palmitic acid, stearic acid, oleic acid, linoleic acid, linolenic acid and arachidonic acid. Preferred are unsaturated higher fatty acids having 1 to 4 double bonds and 18 to 20 carbon atoms such as oleic acid, linoleic acid, linolenic acid and arachidonic acid and most preferred is oleic acid. The above listed higher fatty acids may be applied alone or in combination. It is advised that the content of the fatty acids be set at a level not less than 5 % by weight (based on the total weight of the liposome material and the fatty acid) and controlled to that extent at which the phospholipid does not form a micelle. When the amount of the higher fatty acid is 5 % by weight or more, and preferably 10 % by weight or more, then the resultant liposome indicates the intended excellent affinity to the living body, stability and the property of causing the material contained in the liposome to be released into the living body at a desired slow rate. Unless the phospholipid is applied at a concentration ensuring a lameller structure, the liposome fails to hold, for example, a medical material. If the higher fatty acid is added in an increased amount, then the phospholipid will constitute a micelle in which the higher fatty acid is centered, thereby failing to form liposome. The content of the higher fatty acid at this state varies with various conditions, but generally is about 30 % by weight. Unless, therefore, the higher fatty acid is applied at a smaller content than the above-defined level, it is impossible to provide a desired liposome. In such case, a liposome will prominently decrease in the capacity of holding a medical material or in an extreme case utterly fail to sustain the medical material. It is preferred that the higher fatty acid be used in an amount of 15 % by weight or less.

It is possible to add sterols such as cholesterol or tocopherol to the liposome film of this invention in order to elevate the strength of the film. Further fro the adjustment of the slow release of a medical material contained in the liposome film, it is possible to add a negative charge-imparting material, for example, phosphatidic acid or dicetyl phosphate. The presence of such materials prevent the liposome film from being broken. The negative charge-imparting material imparts negative charge to the liposomes and the resultant liposomes are repelled due to the negative charge and prevented from being floculated and fused together.

The amounts of the sterols and the negative charge-imparting material are each less than 35 % by weight (based on the total weight of the liposome film material) and vary dependent on the composition of the liposome film material. For example, egg yolk lecithin, cholesterol and dicetyl phosphate forms a liposome when used in a molar ratio of 7 : 7 : 2, but does not form a liposome when used in a molar rati of 7 : 7 : 4.

No limitation is imposed on, for example, a medical material held in the liposome of this invention, insofar as the formation of the liposome is not obstructed. The material held in the subject liposome is preferred to be such as that which remains unstable if applied in vitro or in vivo, or that which has to be quickly distributed through a particular internal organ. The material, particularly, a medical material which is to be held in the subject liposome includes, for example, insulin, heparin, urokinase, ubidecarenone, methotrexate, neomycin, bleomycin tetracycline, cytochrome C, asparaginase, and cytosine arabinoside. The materials other than the above listed medical materials, for example, a marker, plasmid, DNA and RNA which prove effective when administered into the living body are not subject to any particular limitation. The material to be administered to the living body is used in a medicinally effective amount.

The liposome itself is manufactured by the already known process. For example, a phospholipid film is prepared by dissolving into a suitable solvent, such as chloroform or ethanol, for example, natural phospholipid, hydrogenated natural phospholipid, at least one higher fatty acid and, as desired, sterols, or a material donating a negative charge and distilling off the solvent from the resultant solution. The film so obtained is dissolved in a suitable solvent, such as chloroform or ether to obtain a solution to which an aqueous solution of the medicine is added. The resultant mixture is vigorously agitated and is preferably subjected to an ultrasonic treatment to obtain a uniform dispersion. The solvent is distilled off from the dispersion to obtain a medicine-hoding liposome. This liposome retains such a medicine and the other effective material in a manner taken in the film structure so as to be administered in the living body. In this connection it is to be noted that the diameter of the liposome so obtained ranges from 0.03 μm to 2.0 μm, though varying dependent upon the formation conditions.

The liposome so obtained is cleaned by a physiologically acceptable aqueous solution, as required, such as a physiological saline solution and then mixed with the aqueous solution of the above-mentioned high molecular material. The mix ratio is 1 ~ 50 V/V %.

Thereafter, the mixture is irradiated with a γ-ray to perform a treatment for insolubilization, causing the liposome to be retained in a manner taken in the matrix and thus obtaining a white-colored gel-like material.

The liposome formulation or composition so obtained is used, directly as a medicine, in the form of gel and, after freeze-dried and powdered, may be properly prepared in the form of pellets, suspension, tablets, capsules, granules, powders. The liposome formulation of this invention can be administered to the living body orally or parenterally, for example, by injection.

The liposome formulation or composition may be freeze-dried under the conventional conditons. For example, it is preferred that the liposome formulation be freeze-dried at -20 to -80 ° C followed by sublimating the ice under a vacuum of 0.3 toor or less.

This invention will be further explained below in connection with the following Examples and Controls.

Example 1

32 mg of lecithin, 15.4 mg of cholesterol and 5 mg of olic acid were dissolved in 3 mℓ of chloroform and charged into an eggplant-like flask with a volume of 50 mℓ. 3 mℓ of isopropylether was added to the resultant mixture, followed by being agitated thoroughly. 1 mℓ of an aqueous solution containing 10,000 units of urokinase was added to the resultant mixture. The mixture so obtained was ultrasonically treated in a water-bath type ultrasonic cleaning machine for 5 minutes at 4 ° C to yield a uniform dispersion. Then, the solvent was distilled off from the dispersion with the use of a rotary evaporator until a gel was formed in the water bath of 40 ° C. 5 mℓ of a physiological saline solution was added to the formed gel and agitated. The solvent was continuously distilled off for 10 minutes to obtain a liposome suspension. The suspension was centrifugally removed at 100,000 G and cleaned twice with the physiological saline solution to obtain pellets. The pellts were suspended in the physiological saline solution to remove germs, obtaining a solution of the liposome with urokinase taken therein.

32 V/N % solution of the liposome were mixed with 10 W/V % of polyvinyl alcohol containing 1 W/V % methylenebisacrylamide and the mixture was irradiated at room temperature with a 3 Mard γ-ray to effect an insolubilization treatment, obtaining a white-colored gel-like material. Then, the gel-like material was freeze-dried to give a liposome formulation. Stability tests were conducted in vivo and in vitro with the above-mentioned liposome of its own as Control.

Experiment 1

Tests as to the stability in vitro of a liposome formuation

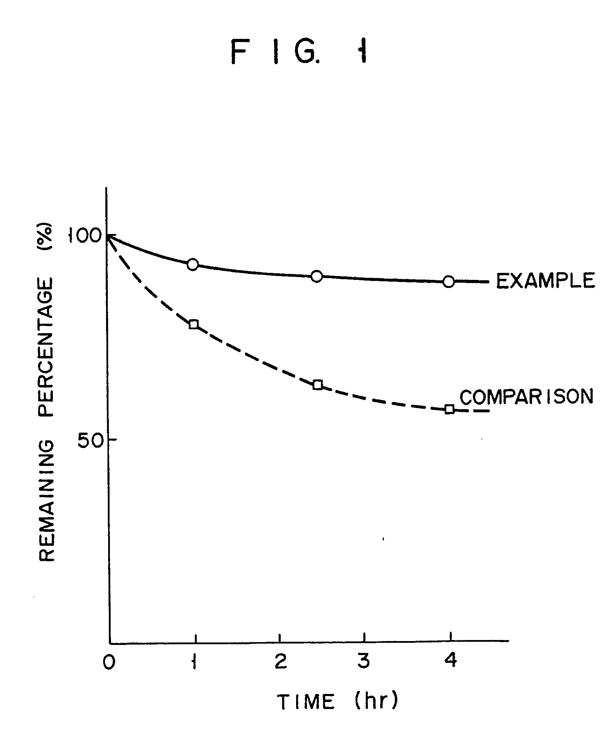

A liposome formulation prepared according to the Example 1 was suspended in the first liquid (artificial gastric juice), the second liquid (artificial intestinal juice) regulated by the Tenth Revised Japanese Pharmacopoeia Destruction Testing Method and 90 % serum of a rabbit, and incubated at 37 ° C, and a remaining percentage of the medicine in the liposome was examined with a varying time, using the synthetic substrate S-2444 method. The results are shown in Figs. 1, 2 and 3.

Circles in Figs. 1, 2 and 3 correspond to the liposome formulation obtained in accordance with Example 1 and squares in these Figures correspond to the sole liposome formulation of Control.

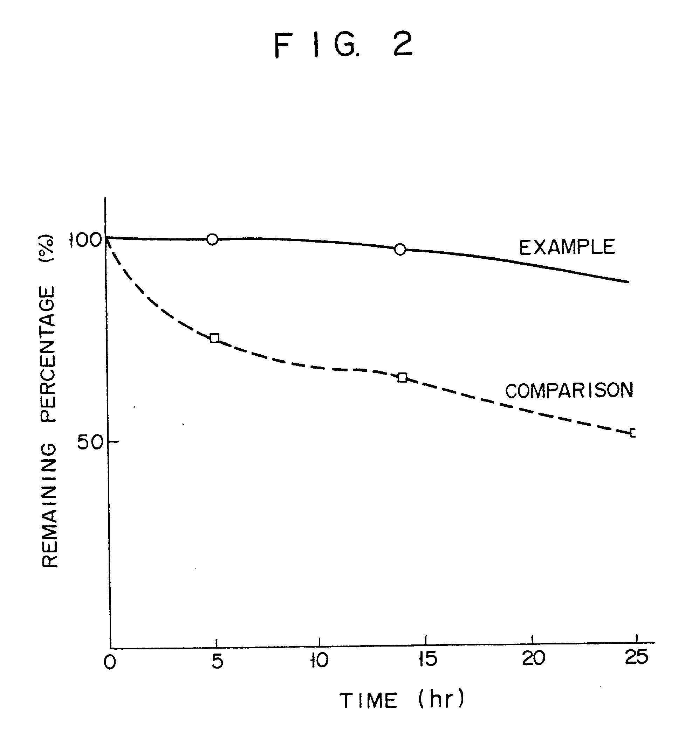

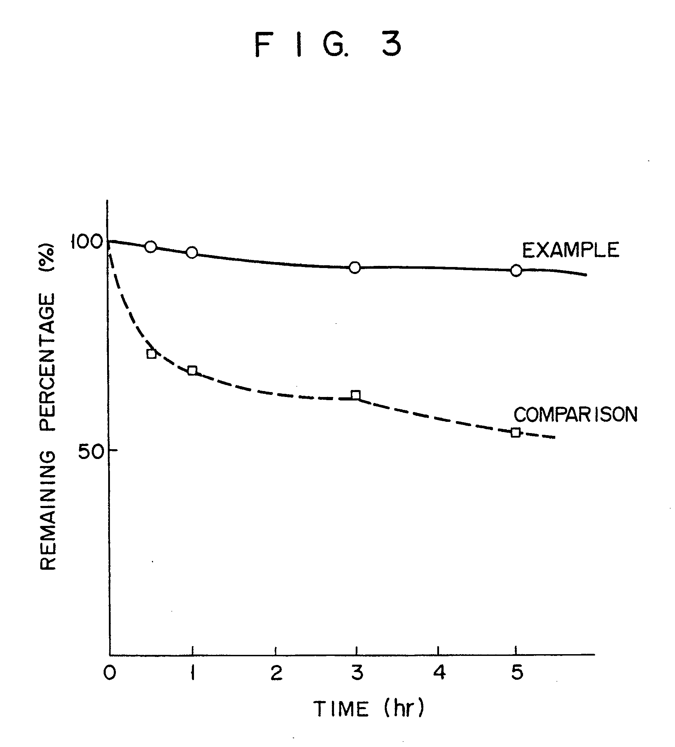

Fig. 1 shows the stability in the first liquid. It has been confirmed that the liposome formulation of this invention reveals an enhanced stability over the liposome of Control. Fig. 2 shows the stability in the second liquid and it has been found that the liposome formulation of this invention manifests an enhanced stability over that of Control. Fig. 3 shows the stability in the rabbit serum and it has been evident that the liposome formulation of this invention shows an enhanced stability over that of Control.

Experiment 2

Tests as to the stability in vivo of a liposome formulation

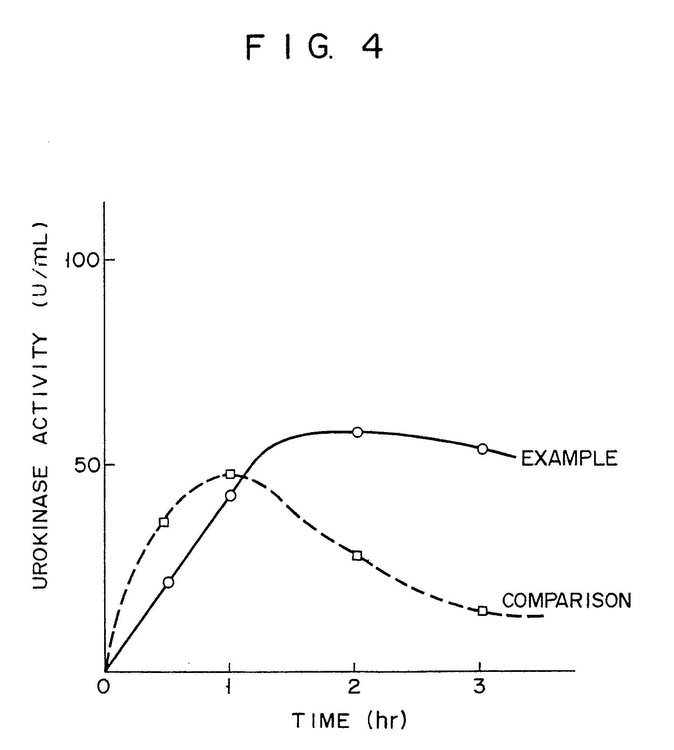

1,000 units of the liposome formulation prepared in Example 1 were orally given in a Wister male rat with a body weight of 200 to 250 g and a blood sample was collected 30, 60, 120 and 180 minutes after administration and the urokinase activity in the plasma was examined using a synthetic substrate S-2444 method, the result of which is shown in Fig. 4.

The circles and squares in Fig. 4 have the same meaning as these in Figs. 1 to 3. From Fig. 4 it is evident that the liposome of this invention reveals an excellent stability in the blood collected, and exhibits an efficacy which has not been obtained in the prior art.

According to the method of this invention as set out above, a liposome supporting a material to be administered is entrapped in the matrix of a crosslinked high molecular material having a three-dimensional network structure obtained through the crosslinking of the material. Therefore, the release of such material in the liposome is satisfactorily sustained in the living body, thus maintaining a stable efficacy over a long period of time.

If, in this case, an uncrosslinked high molecular material is used as such material, it is dissolved in the living body until it reaches a target site (tissue) in the living body where it is absorbed, resulting in a possible destruction of the liposome. However, no such inconvenience occurs when it is crosslinked.

Furthermore, the crosslinked material, if having a hydrophilic groups, provides an adequate compatibility to the living body with the result that it is less likely to be recognized as a foreign material by the living body.

By performing the crosslinking of the high molecular material by irradiating it with a radiation, such as $\gamma$-ray, it is possible to form the matrix without causing any injury to the liposome film or without giving any toxicity to the living body.

## Claims

1. A method of preparing a liposome composition including a cross-linked material having a three-dimensional network structure, and a liposome enclosing a medicine to be administered into a living body, said method comprising:

   preparing an aqueous solution of a cross-linkable material;

   mixing a liposome enclosing a medicine with said aqueous solution; and

   irradiating the resulting mixture with gamma-rays, thereby cross-linking said cross-linkable material to have a three-dimension network structure and physically entrapping said liposome within said network structure.

2. A method according to claim 1, characterized in that said cross-linkable material comprises a hydrophilic group.

3. A method according to claim 2, characterized in that said cross-linkable material is selected from the group polyacrylamide, poly(2-hydroxyethylmethacrylate), polymethacrylic acid, poly(N,N-dimethylaminoethylmethacrylate), polyethylene oxide, polyvinyl alcohol, gelatin, polyvinylpyrrolidone, polysaccharides, sodium alginate, collagen fibrin, silicone, collodion and derivatives thereof.

4. A method according to claim 1, characterized in that the liposome is formed of a film material mainly containing phospholipids.

5. A method according to claim 4, characterized in that said film material contains a higher fatty acid.

6. A method according to claim 4, characterized in that said film material contains sterols.

7. A method according to claim 1, characterized in that said medicine to be administered into a living body is a plasmid, DNA or RNA.

8. A method according to claim 1, characterized in that the liposome has a particle size of 0.03 $\mu$m to 2.0 $\mu$m.

9. A method according to claim 1, characterized in that said cross-linkable material is pharmaceutically acceptable.

10. A method according to claim 1, characterized in that said medicine to be administered into the living body is present in a medicinally effective amount.

11. A method according to claim 1, characterized in that said liposome is held between each lattice which is the minimum unit constituting said network structure, and said liposome has a particle size larger than the lattice.

## Revendications

1. Procédé de préparation d'une composition de liposomes, comprenant un matériau réticulé présentant une structure en réseau tridimensionnel, et des liposomes renfermant des médicaments à administrer à un être vivant, ledit procédé comprenant :

   la préparation d'une solution aqueuse d'un matériau réticulable ;

   le mélange de liposomes renfermant un médicament avec ladite solution aqueuse ; et

   l'irradiation du mélange résultant par des rayons gamma, ce qui provoque la réticulation dudit matériau réticulable en une structure en réseau tridimensionnel et le piègeage physique desdits liposomes au sein de ladite structure en réseau.

2. Procédé conforme à la revendication 1, **caractérisé** en ce que le matériau réticulable comporte des groupes hydrophiles.

3. Procédé conforme à la revendication 2, **caractérisé** en ce que ledit matériau réticulable est choisi parmi un polyacrylamide, un poly-

(méthacrylate de 2-hydroxyéthyle), un poly-(acide méthacrylique), un poly(méthacrylate de N,N-diméthylaminoéthyle), un poly-(oxyéthylène), un poly(alcoolvinylique), une gélatine, un poly(vinylpyrrolidone), les polysaccharides, un alginate de sodium, une fibrine de collagène, une silicone, un collodion, et leurs dérivés.

4. Procédé conforme à la revendication 1, **caractérisé** en ce que les liposomes sont formés d'un matériau filmogène contenant principalement des phospholipides.

5. Procédé conforme à la revendication 4, **caractérisé** en ce que ledit matériau filmogène contient un acide gras supérieur.

6. Procédé conforme à la revendication 4, **caractérisé** en ce que ledit matériau filmogène contient des stérols.

7. Procédé conforme à la revendication 1, **caractérisé** en ce que ledit médicament à administrer à un être vivant est un plasmide, un ADN ou un ARN.

8. Procédé conforme à la revendication 1, **caractérisé** en ce que les liposomes présentent une dimension de particule valant de 0,03 $\mu$m à 2,0 $\mu$m.

9. Procédé conforme à la revendication 1, **caractérisé** en ce que ledit matériau réticulable est acceptable du point de vue pharmaceutique.

10. Procédé conforme à la revendication 1, **caractérisé** en ce que ledit médicament à administrer à un être vivant est présent en une quantité médicalement efficace.

11. Procédé conforme à la revendication 1, **caractérisé** en ce que lesdits liposomes sont maintenus entre les mailles, qui sont les unités minimales constituant ladite structure en réseau, et en ce que lesdits liposomes présentent une dimension de particule plus grande que celle des mailles.

## Patentansprüche

1. Verfahren zur Herstellung einer Liposommasse einschließlich eines vernetzten Materials mit einer dreidimensionalen Netzwerkstruktur und eines Liposoms, in dem eine an einen lebenden Körper zu verabereichende Medizin untergebracht ist, durch
Zubereiten einer wäßrigen Lösung eines vernetzbaren Materials;
Vermischen eines Liposoms, in dem eine Medizin eingeschlossen ist, mit der wäßrigen Lösung; und Bestrahlen des erhaltenen Gemischs mit $\gamma$-Strahlung zur Vernetzung des vernetzbaren Materials unter Ausbildung einer dreidimensionalen Netzwerkstruktur und physikalischem Einschluß des Liposoms in der Netzwerkstruktur.

2. Verfahren nach Anspruch 1, dadurch gekennzeichnet, daß das vernetzbare Material eine hydrophile Gruppe aufweist.

3. Verfahren nach Anspruch 2, dadurch gekennzeichnet, daß das vernetzbare Material aus der Gruppe Polyacrylamid, Poly-(2-hydroxyethylmethacrylat), Polymethacrylsäure, Poly-(N,N-dimethylaminoethylmethacrylat), Polyethylenoxid, Polyvinylalkohol, Gelatine, Polyvinylpyrrolidon, Polysaccharide, Natriumalginat, Kollagenfibrin, Silikon, Collodion und Derivate derselben ausgewählt ist.

4. Verfahren nach Anspruch 1, dadurch gekennzeichnet, daß das Liposom aus einem hauptsächlich Phospholipide enthaltenden Filmmaterial gebildet ist.

5. Verfahren nach Anspruch 4, dadurch gekennzeichnet, daß das Filmmaterial eine höhere Fettsäure enthält.

6. Verfahren nach Anspruch 4, dadurch gekennzeichnet, daß das Filmmaterial Sterine enthält.

7. Verfahren nach Anspruch 1, dadurch gekennzeichnet, daß die einem lebenden Körper zu verabreichende Medizin aus einem Plasmid, DNA oder RNA besteht.

8. Verfahren nach Anspruch 1, dadurch gekennzeichnet, daß das Liposom eine Teilchengröße von 0,03 $\mu$m bis 2,0 $\mu$m aufweist.

9. Verfahren nach Anspruch 1, dadurch gekennzeichnet, daß das vernetzbare Material pharmazeutisch akzeptabel ist.

10. Verfahren nach Anspruch 1, dadurch gekennzeichnet, daß die dem lebenden Körper zu verabreichende Medizin in einer medizinisch wirksamen Menge vorhanden ist.

11. Verfahren nach Anspruch 1, dadurch gekennzeichnet, daß das Liposom zwischen jedem Gitter, das die die Netzwerkstruktur bildende Mindesteinheit darstellt, gehalten wird und eine das Gitter übersteigende Teilchengröße auf-
weist.

# F I G. 1

# F I G. 2

# F I G. 3

# F I G. 4